# EUROPÄISCHE PATENTSCHRIFT

(11) **EP 0 765 158 B1**
(45) Veröffentlichungstag und Bekanntmachung des Hinweises auf die Patenterteilung: **04.11.1998**
(21) Anmeldenummer: 95921828.0
(22) Anmeldetag: 03.06.1995
(51) Int. Cl.: A61K 9/20, A61K 9/00, A61K 9/70

(54) **WIRKSTOFFTRÄGER ZUR KONTROLLIERTEN FREISETZUNG VON WIRKSTOFFEN IM GASTROINTESTINALTRAKT MIT VERZÖGERTER PYLORUSPASSAGE**
ACTIVE SUBSTANCE CARRIER FOR THE CONTROLLED RELEASE OF ACTIVE SUBSTANCES IN THE GASTRO-INTESTINAL TRACT WITH DELAYED PASSAGE THROUGH THE PYLORUS
EXCIPIENT DESTINE A LA LIBERATION CONTROLEE DE PRINCIPES ACTIFS DANS LE TRACTUS GASTRO-INTESTINAL AVEC PASSAGE RETARDE DANS LE PYLORE

(30) Priorität: 07.06.1994 DE 4419818
(43) Veröffentlichungstag der Anmeldung: 02.04.1997
(73) Patentinhaber: LTS LOHMANN Therapie-Systeme GmbH, 56567 Neuwied (DE)
(72) Erfinder: ASMUSSEN, Bodo, D-56170 Bendorf-Sayn (DE); CREMER, Karsten, D-53119 Bonn (DE); MÜLLER, Walter, D-56565 Neuwied (DE)
(74) Vertreter: Flaccus, Rolf-Dieter, Dr.
(86) Internationale Anmeldenummer: EP9502121
(87) Internationale Veröffentlichungsnummer: WO9533449

(56) Entgegenhaltungen:
- EP-A- 0 202 159
- EP-A- 0 415 671
- FR-A- 2 335 206

## Beschreibung

Die Erfindung betrifft die Verwendung eines bahnförmigen, folienartigen, mit Öffnungen ausgebildeten, und mit Wirkstoff beladenen Substrates.

Wirkstoffträger bzw. Arzneiformen mit verlängerter Verweildauer im Magen sollen eine lokale Therapie bei Magenkrankheiten ermöglichen. Zusätzlich erlauben sie die Freisetzung von Wirkstoffen über einen insgesamt längeren Zeitraum als konventionelle perorale Arzneiformen, so daß eine Verringerung der Einnahmefrequenz erzielt werden kann.

Eine verlängerte Verweildauer im Magen besitzen Arzneiformen, die entweder eine besonders geringe Dichte aufweisen und auf der Magenflüssigkeit flotieren oder solche, die aufgrund ihrer Größe oder Sperrigkeit den Pylorus nicht passieren können.

Flotierende Arzneiformen sind zum Beispiel solche, die einen hohen Anteil lipophiler Substanzen mit geringer Dichte aufweisen (DE 26 11 041). Ferner wurde beschrieben, wie durch eine Vielzahl kleiner Lufteinschlüsse eine Retardtablette oder -kapsel zum Flotieren gebracht werden kann (EP-A 0 297 978, DE-A 38 03 482). Schließlich lassen sich gaserzeugende Substanzen oder Gemische wie zum Beispiel CO₂-erzeugende Brausemischungen in eine umhüllte Arzneiform einarbeiten, was gleichzeitig mit einer Expansion einer solchen Vorrichtung nach der Applikation verbunden ist (US 4 996 058).

Ein entscheidender Nachteil der flotierenden Arzneiformen ist die Unzuverlässigkeit ihrer Gastroretentivität. Solange Ihre Pyloruspassage nicht zusätzlich durch ihre Größe behindert ist (EP-A 0 308 904), findet eine Flotation nur bei aufrecht sitzenden oder stehenden Personen statt. Bei liegenden Personen kann der Pylorus sehr leicht mit der Oberfläche des Magensaftes in Kontakt kommen, wobei die flotierende Arzneiform bevorzugt in den Dünndarm transportiert wird (A.J. Moës, Crit. Rev. Therap. Drug Carrier Syst. 10, 143, 1993). Außerdem setzt das Flotieren solcher Vorrichtungen die Anwesenheit einer Mindestmenge von Magensaft voraus, wovon man aber bei Patienten nicht immer ausgehen kann.

Arzneiformen, die aufgrund ihrer Größe oder Sperrigkeit im Magen retiniert werden, sind ebenfalls seit längerem bekannt. Da die Arzneiform verschluckt werden muß und deshalb zunächst eine bestimmte Maximalausdehnung nicht überschreiten darf, wurden verschiedene Mechanismen beschrieben, mit denen nach der Applikation eine Vergrößerung der Vorrichtung im Magen erfolgen sollte. Dies kann zum Beispiel durch die Erzeugung einer Gasphase nach dem Kontakt mit wäßriger Flüssigkeit in der Vorrichtung (US 4 996 058), aber auch durch die Quellung hydrophiler Komponenten im Magensaft (EP 0 425 154, US 5 147 646, EP 0 310 326, US 4 207 890, US 4 434 153) entstehen. Diese Arzneiformen haben vor allem den Nachteil, daß sie entweder nicht genügend Festigkeit besitzen, um den kontraktilen Kräften der Magenwandmuskulatur zu widerstehen oder - wenn sie diese Festigkeit besitzen - die Gefahr in sich bergen, zu einer unerwünschten und evtl. gefährlichen Obstruktion des Pylorus führen zu können, durch die der Weitertransport des übrigen Mageninhaltes unterbunden wird.

Bekannt sind verschieden geformte sperrige wirkstofftragende Vorrichtungen, welche zur Applikation zunächst komprimiert oder kontrahiert vorliegen. Dabei wird der komprimierte bzw. kontrahierte Zustand in der Regel durch Umhüllungen wie zum Beispiel Kapseln bis zum Zerfall derselben im Magensaft fixiert. Nach dem Zerfall der Umhüllung bewirken Rückstellkräfte oder der Quellungsdruck hydrophiler Komponenten das Zurückformen der sperrigen Gebilde (US 4 735 804, EP 0 202 159, US 5 002 772, EPA 0 415 671). Solange diese eine genügende mechanische Stabilität aufweisen, um der Magenkontraktion gegenüber resistent zu sein, eignen sie sich potentiell am besten als gastroretentive Arzneiformen, da ihre Pyloruspassage zuverlässig unterbunden wird, ohne die Entleerung des übrigen Mageninhaltes zu verhindern.

Im Gegensatz zu den Konstruktionsmerkmalen verschiedener gastroretentiver Systeme werden im Stand der Technik kaum fertigungstechnische Aspekte beschrieben. Aufgrund der komplexen Formgebung der bekannten gastroretentiven Arzneiformen ist die industrielle Serienfertigung solcher Systeme als äußerst aufwendig und kostspielig anzusehen. Kontinuierliche Verfahren sind bisher nicht beschrieben worden. Gastroretentive Arzneiformen enthalten oft verschiedene Komponenten, die jeweils entweder der Formerhaltung oder der Wirkstofffreisetzung dienen. Es existieren auch keine pharmazeutischen Prüfmethoden, mit denen die Qualität dieser Arzneiformen während der Herstellung sichergestellt werden kann. Große und sperrige Gebilde mit hoher mechanischer Stabilität sind nicht nur schwer zu fertigen; sie besitzen zudem den Nachteil, daß sie im Magen zu Irritationen führen können.

Der Erfindung liegt die Aufgabe zugrunde, einen Wirkstoffträger zur kontrollierten Freisetzung von Wirkstoffen in den Gastrointestinaltrakt mit verzögerter Pyloruspassage anzugeben, welcher die vorgenannten Nachteile und Schwierigkeiten vermeidet bzw. überwindet, bei einer lokalen Therapie des Magens beziehungsweise der Magenschleimhaut eine bedeutend verlängerte Verweildauer im Magen unter Freisetzung von Wirkstoffen ermöglicht, die orale Applikation möglichst weitgehend erleichtert und sich für eine Serienherstellung mit wirtschaftlichen Mitteln eignet.

Zur Lösung der Aufgabe wird mit der Erfindung eine Verwendung von bahnförmigem, mit Öffnungen ausgebildetem Wirkstoffträger und mit Wirkstoff beladenen Substrates als Ausgangsmaterial zur Herstellung eines Wirkstoffträgers zur kontrollierten Freisetzung von Wirkstoffen im Gastrointestinaltrakt mit verzögerter Pyloruspassage vorgeschlagen.

Das Wirkstoffträger-Material ist in verschiedener Hinsicht sehr vorteilhaft. Er ermöglicht eine wirtschaftliche Fertigung unter Einsatz bekannter, kontinuierlicher Verfahren zur Verarbeitung von bahnförmigem, folienartigem Material wie beispielsweise Auf- und Abwickeln, Beschichten und Stanzen sowie Umhüllen mit einem den Zusammenhalt fördernden Material. Die hiermit herstellbaren gastroretentiven Ausbildungsformen weisen weitere vorteilhafte Eigenschaften auf. Beispielsweise sind wegen der folienförmigen Ausgestaltung des Wirkstoffträgers Irritationen der Magenschleimhaut, wie sie bei bekannten dreidimensional sperrigen Gebilden auftreten, die aufgrund ihrer Größe oder Sperrigkeit im Magen retiniert werden, nicht oder nur in äußerst geringem Ausmaß zu erwarten. Insbesondere bei ausreichender mechanischer Stabilität der nach Erosion der Umhüllung entfalteten Vorrichtung im Magensaft ist eine hohe Zuverlässigkeit hinsichtlich der Gastroretentivität zu erwarten. Bei einer erreichbaren Mindestausdehnung von 5 cm² übertrifft der Wirkstoffträger die Größe der Öffnung des menschlichen Pylorus, wobei eine maximale Ausdehnung auf bis zu 8 cm² mit noch größerer Sicherheit die Pyloruspassage verhindert. Auch läßt sich die beabsichtigte Verweildauer des Wirkstoffträgers im Magen besser einstellen als bei den meisten bisherigen Systemen, bei welchen die Pyloruspassage eintritt, wenn der Magenmuskulatur eine genügende Zerkleinerung der Vorrichtung gelungen ist. Weil das Ausmaß der Magenkontraktionen von einer großen Zahl von Faktoren abhängt, die in der Praxis kaum kontrolliert werden können, wie beispielsweise dem Aktivitätszustand des autonomen Nervensystems, insbesondere des Parasymphatikus, dürfte die Retentionsdauer der bisher vorgeschlagenen Arzneiformen im Anwendungsfall mit einer großen interindividuellen Variabilität behaftet sein. Die Retentionszeit des erfindungsgemäßen Wirkstoffträgers läßt sich dagegen mit erheblich größerer Sicherheit über die galenische Formulierung einstellen: Die Komposition bestimmt die Geschwindigkeit, mit welcher die Vorrichtung im Magensaft - vorwiegend durch Erosion - die Eigenschaften verliert, welche ihre Retention bewirken.
Dadurch, daß der Wirkstoffträger mit Öffnungen ausgebildet ist, wird der Gefahr einer unbeabsichtigten Pylorus-Obstruktion entgegengewirkt. Im Normalfall ermöglicht der Pylorus die Passage von flüssigem Material geringer bis mittlerer Viskosität sowie von partikulärem Material mit einer Korngröße bis zu etwa 2 mm. Wenn der Wirkstoffträger beispielsweise Öffnungen von 3 mm Durchmesser oder mehr aufweist, ist sichergestellt, daß eine unerwünschte Pylorusobstruktion nicht eintreten kann. Selbst wenn die Vorrichtung sich über die Pylorusöffnung legen sollte, würde sie lediglich einen Siebeffekt bewirken.

Eine Ausgestaltung der Erfindung sieht vor, daß der Wirkstoffträger mindestens einen Wirkstoff enthält, der eine lokale Wirkung im Magen entfaltet. Es kann sich dabei um einen Wirkstoff handeln, der bevorzugt von der Magenschleimhaut resorbiert wird. Es kann sich aber auch um einen Wirkstoff handeln, für den es im Bereich des oberen Dünndarm eine Resorptionsfenster gibt. Damit ist der Erfindungsgegenstand auch für die Verabreichung solcher Wirkstoffe geeignet, die ihre Wirkung im oberen Dünndarmabschnitt entfalten. Hierfür waren bisher herkömmliche perorale Retardformen ungeeignet, weil sie einen großen Teil der Dosis erst dann abgeben, wenn diese den oberen Dünndarmbereich bereits verlassen haben. Mit dem erfindungsgemäßen Wirkstoffträger kann sichergestellt werden, daß freigesetzter Wirkstoff in gelöster Form gleichmäßig über eine längere Zeit dem oberen Dünndarm zugeführt wird.

Die Erfindung bringt mehrere therapeutische Vorteile mit sich. Zum einen ermöglicht sie eine gegenüber herkömmlichen Arzneiformen wesentlich verbesserte lokale Therapie von Magenkrankheiten. Dazu gehören insbesondere die Hyperazidität, mikrobielle Infektionen, die Gastritis und das Ulcus. Zur Behandlung dieser und anderer Magenkrankheiten stehen wirksame Arzneistoffe zur Verfügung, deren Effektivität beziehungsweise therapeutischer Index dadurch gesteigert wird, daß sie mit dem erfindungsgemäßen Wirkstoffträger verabreicht werden. Damit wird ein besonders hoher Anteil der applizierten Wirkstoffdosis unmittelbar mit dem erkrankten Gewebe in Kontakt gebracht. Die Dauer der lokalen Exposition mit wirksamen Arzneistoffkonzentrationen wird erhöht. Beispiele für solche Wirkstoffe sind mineralische Antacida, H₂-Rezeptorenblocker wie Cimetidin, Ranitidin, Famotidin, Nizatidin, Roxatidin und ihre Salze, Muscarinrezeptorenblocker wie Pirenzipin, sogenannte Protonenpumpen wie Omeprazol und Misoprostol; Arzneistoffe mit Wirksamkeit gegen Helicobacter pylorii und andere mikrobielle Noxen, Proglumid und Carbenoxolon.

Eine Ausgestaltung sieht vor, daß der Wirkstoffträger zusätzlich gasförmige Stoffe, gaserzeugende Stoffe bzw. Stoffgemische oder flüssige bzw. feste Stoffe und/oder deren Gemische mit einer relativen Dichte <1 enthält. Damit wird die Retention des Wirkstoffträgers im Magen unterstützt, indem dieser zusätzlich zu seiner Flächengröße zu einer flotierenden Vorrichtung wird.

Weiter sieht die Erfindung vor, daß der Wirkstoffträger aus einem in biologischen Flüssigkeiten erodierbaren Material besteht oder solches enthält. Die Zusammensetzung des verwendeten Materials bestimmt im wesentlichen die Verweildauer der gastroretentiven Vorrichtung im Magen. Vorzugsweise besteht es aus einem oder mehreren physiologisch unbedenklichen Polymeren und weiteren pharmazeutischen Hilfsstoffen, wie z.B. Weichmachern, Netzmitteln, Hydrophilisierungsmitteln, Stabilisatoren, Farbstoffen, Formtrennmitteln, Puffersalzen usw. Beispiele für die zu verwendenden Polymere sind Polysaccharide, wie Gummen, Stärke oder Cellulosederivate, Polyacrylate und -methacrylate, Polylaktide, Polyglykolide, Polyoxyethylene und Polyoxypropylene, Proteine, Polyvinylalkohol, Polyvinylacetat, Polyvinylchlorid oder Polvvinylpyrrolidon, Siliconelastomere sowie Copolymere. Mit geeigneten Mischungen läßt sich eine Erosionsgeschwindigkeit einstellen, mit welcher die angestrebte Retentionsdauer im Magen erzielt werden kann. Nach dem Ablauf dieser Zeit hat eine erfindungsgemäß hergestellte Vorrichtung ihre mechanische Stabilität soweit verloren, daß sie durch Magenkontraktion zerkleinerbar ist und somit den Pylorus passieren kann. Nach Ablauf dieser Zeit hat ein erfindungsgemäß hergestellter Wirkstoffträger seine mechanische Stabilität soweit verloren, daß er durch Magenkontraktionen zerkleinerbar ist und somit den Pylorus passieren kann.

Erfindungsgemäß herstellbare Wirkstoffträger werden durch Rollen oder Falten in ihren Abmessungen derart verändert, daß sie sich für eine perorale Applikation eignen. Dies setzt voraus, daß das folienartige Material flexibel und nicht spröde ist. Nach dem Aufrollen oder Falten werden die Vorrichtungen bevorzugt mit einer geeigneten Umhüllung versehen, durch welche sie in diesem Zustand bis zur Applikation fixiert werden. Eine Hartgelatinekapsel stellt z.B. eine solche Umhüllung dar, jedoch ist jede Umhüllung geeignet, welche die Vorrichtung in gerolltem oder gefaltetem Zustand fixiert, im Magensaft zerfällt und physiologisch unbedenklich ist.

Insgesamt zeichnet sich der Wirkstoffträger gegenüber dem Stand der Technik durch eine Fülle von Vorteilen aus. Dazu gehört, daß mit dem Wirkstoffträger die Möglichkeit gegeben wird, die Einnahmefrequenz bei einer Dauertherapie über das Maß der herkömmlichen Retardformen hinaus herabzusetzen. Retard-Arzneimittel sind in der Dauertherapie in der Regel dann sinnvoll, wenn der Wirkstoff schnell aus dem Körper eliminiert wird, d.h. mit einer Eliminationshalbwertzeit von weniger als etwa 10-20 h. Kann man mit Retardformen die Einnahmefrequenz herabsetzen, so lassen sich große Blutspiegelschwankungen und damit unerwünschte Nebenwirkungen vermeiden und die Patienten-Compliance verbessern. Bei der Verwendung des erfindungsgemäßen Wirkstoffträgers läßt sich die Einnahmefrequenz vieler Wirkstoffe auf eine tägliche Dotierung reduzieren, was einen weiteren Fortschritt in der Therapiesicherheit bedeutet.

## Patentansprüche

1. Verwendung eines bahnförmigen, folienartigen, mit Öffnungen ausgebildeten, und mit Wirkstoff beladenen Substrates als Ausgangsmaterial zur Herstellung eines folienartigen, mit Öffnungen ausgebildeten Wirkstoffträgers zur kontrollierten Freisetzung von Wirkstoffen im Gastrointestinaltrakt mit verzögerter Pyloruspassage.

2. Ausgangsmaterial zur Herstellung eines Wirkstoffträgers nach Anspruch 1, dadurch gekennzeichnet, daß es mindestens einen Wirkstoff enthält, der eine lokale Wirkung im Magen entfaltet.

3. Ausgangsmaterial zur Herstellung eines Wirkstoffträgers nach Anspruch 1 oder 2, dadurch gekennzeichnet, daß es mindestens einen Wirkstoff enthält, der bevorzugt von der Magenschleimhaut resorbiert wird.

4. Wirkstoffträger nach Anspruch 1, dadurch gekennzeichnet, daß er mindestens einen Wirkstoff enthält, für den es im Bereich des oberen Dünndarms ein Resorptionsfenster gibt.

5. Wirkstoffträger nach einem oder mehreren der Ansprüche 1-4, dadurch gekennzeichnet, daß er zusätzlich gasförmige Stoffe, gaserzeugende Stoffe bzw. Stoffgemische oder flüssige bzw. feste Stoffe und/oder deren Gemische mit einer relativen Dichte <1 enthält.

6. Wirkstoffträger nach einem oder mehreren der Ansprüche 1-5, dadurch gekennzeichnet, daß er aus einem in biologischen Flüssigkeiten erodierbaren Material besteht oder solches enthält.

7. Wirkstoffträger nach einem oder mehreren der Ansprüche 1-6, dadurch gekennzeichnet, daß er mit einer oral applikationsfähigen Kompaktform als Rolle oder Faltkomprimat ausgebildet ist.

8. Wirkstoffträger nach einem oder mehreren der Ansprüche 1-7, dadurch gekennzeichnet, daß er in seiner Kompaktform von einer Umhüllung, z.B. einer Kapsel, umgeben ist, die im Magensaft rasch zerfällt.

9. Wirkstoffträger nach einem oder mehreren der Ansprüche 1-8, dadurch gekennzeichnet, daß er im entrollten oder entfalteten Zustand eine Fläche von wenigstens 5 cm² aufweist.

10. Verfahren zur Herstellung eines Wirkstoffträgers gemäß einem oder mehreren der Ansprüche 2-9 unter Verwendung von bahnförmigem, mit Öffnungen versehenem, folienartigem Material.

11. Verfahren nach Anspruch 10, dadurch gekennzeichnet, daß es kontinuierlich ausgestaltet ist.

## Claims

1. The use of a web-shaped, sheet-like substrate provided with openings and charged with active substance as starting material for the production of a sheet like active substance carrier, provided with openings, for the controlled release of active substances in the gastrointestinal tract with a delayed pylorus passage.

2. The starting material for the production of an active substance carrier according to claim 1, characterized in that it comprises at least one active substance developing a local action in the stomach.

3. The starting material for the production of an active substance carrier according to claim 1 or 2, characterized in that it comprises at least one active substance which is preferably absorbed by the gastric mucosa.

4. An active substance carrier according to claim 1 characterized in that it comprises at least one active substance for which an absorption window exists in the region of the upper small intestine.

5. The active substance carrier according to one or several of claims 1-4 characterized in that it additionally comprises gaseous substances, gas-producing substances or substance mixtures, or liquid and solid substances and/or their mixtures, which have a relative density of < 1.

6. The active substance carrier according to one or several of claims 1-5 characterized in that it consists of or comprises a material which is erodable in biological liquids.

7. The active substance carrier according to one or several of claims 1-6 characterized in that it is formed with an orally applicable compacted form as a roll or folded compressed article.

8. The active substance carrier according to one or several of claims 1-7 characterized in that its compact form is surrounded by a casing, e.g., a capsule, which disintegrates rapidly in the gastric juice.

9. The active substance carrier according to one or several of claims 1-8 characterized in that it has an area of at least 5 cm² in the unrolled or unfolded state.

10. A process for the production of an active substance carrier according to one or several of claims 2-9 by using web-shaped, sheet-like material provided with openings.

11. The process according to claim 10 characterized in that it is a continuous process.

## Revendications

1. Utilisation d'un substrat en feuille en forme de bande dans lequel sont pratiquées des ouvertures et qui est chargé avec un principe actif, à titre de matière de départ pour la fabrication d'un support en feuille destiné à un principe actif et dans lequel ont été pratiquées des ouvertures, pour la libération contrôlée de principes actifs dans le tractus gastro-intestinal, soumise à un ralentissement au cours du passage dans le pylore.

2. Matière de départ pour la fabrication d'un support de principe actif selon la revendication 1, caractérisée en ce qu'il contient au moins un principe actif qui déploie une activité locale dans l'estomac.

3. Matière de départ pour la fabrication d'un support de principe actif selon la revendication 1 ou 2, caractérisée en ce qu'il contient au moins un principe actif qui est de préférence résorbé par la muqueuse de l'estomac.

4. Support de principe actif selon la revendication 1, caractérisé en ce qu'il contient au moins un principe actif pour lequel une fenêtre de résorption existe dans la zone de l'intestin grêle supérieur.

5. Support de principe actif selon une ou plusieurs des revendications 1 à 4, caractérisé en ce qu'il contient en outre des substances gazeuses, des substances, respectivement des mélanges de substances générant des gaz ou encore des substances liquides, respectivement solides et/ou leurs mélanges, dont la densité relative s'élève à < 1.

6. Support de principe actif selon une ou plusieurs des revendications 1 à 5, caractérisé en ce qu'il est constitué par ou contient une matière manifestant une aptitude à l'érosion dans des liquides biologiques.

7. Support de principe actif selon une ou plusieurs des revendications 1 à 6, caractérisé en ce qu'il est réalisé sous l'aspect d'une forme compacte apte à une administration par voie orale sous forme d'un rouleau ou d'un produit comprimé à l'état replié.

8. Support de principe actif selon une ou plusieurs des revendications 1 à 7, caractérisé en ce qu'il est entouré, dans sa forme compacte, d'un enrobage, par exemple d'une capsule qui se décompose rapidement dans le suc gastrique.

9. Support de principe actif selon une ou plusieurs des revendications 1 à 8, caractérisé en ce qu'il présente, à l'état déroulé ou à l'état déplié, une surface d'au moins 5 cm².

10. Procédé pour la fabrication d'un support de principe actif selon une ou plusieurs des revendications 2 à 9, en utilisant une matière en feuille en forme de bande munie d'ouvertures.

11. Procédé selon la revendication 10, caractérisé en ce qu'il est mis en oeuvre en continu.
